# EUROPEAN PATENT SPECIFICATION

(11) **EP 3 410 925 B1**
(45) Date of publication and mention of the grant of the patent: **07.07.2021**
(21) Application number: 17705969.8
(22) Date of filing: 02.02.2017
(51) Int. Cl.: A61B 5/00, A61B 5/026

(54) **OPTICAL PROBE FOR LOCALIZING AND IDENTIFYING A TARGET TISSUE PRIOR TO HARVESTING A BIOPSY**
OPTISCHE SONDE ZUR LOKALISIERUNG UND IDENTIFIZIERUNG EINES ZIELGEWEBES VOR DER ENTNAHME EINER BIOPSIE
SONDE OPTIQUE POUR LOCALISER ET IDENTIFIER UN TISSU CIBLE AVANT LE PRÉLÈVEMENT D'UNE BIOPSIE

(30) Priority: 05.02.2016 SE 1600030
(43) Date of publication of application: 12.12.2018
(73) Proprietor: FluoLink AB, 582 19 Linköping (SE)
(72) Inventor: Haj-Hosseini, Neda, 585 72 Linköping (SE); Wårdell, Karin, 582 18 Linköping (SE); Richter, Johan, 581 22 Linköping (SE)
(74) Representative: Valea AB
(86) International application number: PCT/SE2017/050083
(87) International publication number: WO 2017/135873

(56) References cited:
- US-A- 5 916 171
- US-A1- 2005 234 315
- US-A1- 2015 148 629
- US-A1- 2015 238 085
- US-B1- 6 537 211
- PETER REJMSTAD ET AL: "A laser Doppler system for monitoring of intracerebral microcirculation", ENGINEERING IN MEDICINE AND BIOLOGY SOCIETY (EMBC), 2013 35TH ANNUAL INTERNATIONAL CONFERENCE OF THE IEEE, IEEE, 28 August 2012 (2012-08-28), pages 1988-1991, XP032463327, ISSN: 1557-170X, DOI: 10.1109/EMBC.2012.6346346
- WARDELL KARIN ET AL: "Relationship Between Laser Doppler Signals and Anatomy During Deep Brain Stimulation Electrode Implantation Toward the Ventral Intermediate Nucleus and Subthalamic Nucleus", OPERATIVE NEUROSURGERY,, vol. 72, no. 2, Suppl Operative, 31 May 2013 (2013-05-31), pages ons127-ons140, XP009521110, ISSN: 2332-4252, DOI: 10.1227/NEU.0B013E31827E5821

## Description

### Technical field

The present invention relates generally to an optical probe with multiparametric features for localizing and identifying a biopsy site in a target tissue i.e. a malignant tumour, prior to harvesting a biopsy, a method and a system, thereof, and use of the same. More particularly, the technical field relates to neurosurgery.

### Background

Brain tumour resection is a challenging task due to the severe consequences that misdiagnosis brings. Diagnosis is commonly done by magnetic resonance imaging (MRI) and computed tomography (CT). When the diagnosis of the tumour type cannot be based on the images alone a biopsy is taken from the suspected tissue. The biopsies are harvested using a stereotactic neurosurgical procedure and a preliminary assessment of malignancy can be obtained within 1-2 hours, and then sent for pathological investigations for accurate assessment which takes up about 2 weeks before an optimal treatment is decided. The risks of this type of operation are hemorrhage (5%), infection and seizure (0.5%). The morbidity rate has been reported to be 7% where about 88% of this number relates to the hemorrhage causes (Kongkham *et al.* 2008). This risk is significantly larger for biopsy procedures compared to a deep brain stimulation (DBS) lead implantation. In DBS-surgery the trajectory towards the implantation region in the brain is usually known. For a biopsy, in principle any trajectory from the cortex towards the tumour, i.e. the biopsy site, is necessary to use. Sometimes also more than one trajectory is necessary and thus with further increased risk for passing vessel structures with possible hemorrhage when the biopsy needle is inserted. This risk can be reduced by using a forward looking guide which measures the microvascular blood flow with laser Doppler flowmetry (LD) before the tissue is touched (Wårdell et al., 2013).

Fluorescence guided resection has shown great benefits in the surgery of malignant tumours using the photosensitizer 5-aminolevulinic acid (5-ALA) to give the surgeon a direct feed-back of the tissue type in the operating room (OR), using surgical microscopes (Stummer et al., 2006). 5-ALA accumulates as protoporphyrin IX (PpIX) in tumour cells. Optical fibers for fluorescence probes have previously been used for biopsy procedures (Pålsson et al., 2003). A hand-held probe for fluorescence measurements during tumour resections has previosuly been used (Haj-Hosseini et al., 2010)

Another problem today is that the obtained biopsy does not always reflect the malignancy of the tumour due to misdiagnosis of the pathologist or an error in the position of where the biopsy was obtained, which means that a new biopsy must be harvested and the whole procedure must be repeated. In the usual routine, several biopsies must be harvested to be able to determine where to cut for removing the tumour. This is very demanding since the patient must be under anesthesia for a longer time and the risk for complications increases. Due to these errors, the final diagnosis might be wrongly placed.

US2002026188 discloses a method and device for performing tissue biopsies by using optical methods for distinguishing between diseased and healthy tissue by for example using primary optically labelled markers or antibodies that bind to an endogenous marker upregulated in tumour tissue.

US6537211 discloses a fluorescence endoscope imaging system for mapping cancerous and precancerous tissues in hollow organs by using auto fluorescence. The method further discloses the use of exogenous markers, such as hematoporphyrin derivatives (HpD) for distinguishing healthy and diseased tissue. The device can be used for imaging the tissue and guide a biopsy in real time.

US 2015/0148629 A1 provides an optical spectroscopy probe for providing optical spectroscopy guidance of a mechanical biopsy procedure, and a tissue biopsy device including an optical spectroscopy probe. The optical spectroscopy probe is positionable in a lumen of a mechanical biopsy device. The probe may enable optical spectroscopy guidance in biopsy procedures, including brain biopsy procedures.

The cost regarding surgical resection of tumours is quite high, more accurate methods would keep costs down and also improve patient safety and shorten the operation times. Moreover, the risk of hemorrhage, brings severe consequences (temporary and permanent neurological deficits). Thus, the methods used today are in need of further clinical evaluation using highly sensitive measurement techniques.

### Summary of the invention

The aim of the present invention is to solve some of the problems of the prior art discussed above.

The present invention provides a probe according to claim 1 with multiparametric features for localizing and identifying tumours, for example brain tumours prior to a biopsy. By using said probe a real-time optical signature of malign tissue is seen through a fluorescence peak on a screen, the signal can be combined with for example a real-time audio signature wherein for example the frequency or volume of the audio signal increase with the fluoresence signal. The real time signal may also be a light or flash light signature wherein the frequency of the signal increase with the fluoresence signal.

The present probe further minimizes the risk for haemorrhagic complications, i.e., reduces the bleeding risk during the deployment and navigation into a target tissue.

The present probe further enables that the biopsy taken from the malign tissue reflects the tumour composition which ensures correct diagnosis and choice of treatment.

Moreover, by using the probe of the present invention the patient safety is increased by shortening the procedure of performing a biopsy, identifying correct coordinates for removing the tumour and also identify the correct tissue type for treatment.

The healthcare cost is reduced due to reduced time needed for surgery and health care professionals, decreased risk for misdiagnosis, reduced risk for the need of repeating the procedure for harvesting a biopsy for diagnosis, before removing the tumour by surgery. The present invention provides a probe (1) according to claim 1.

The probe is suitable for intracerebral guidance prior to stereotactic tumour biopsy.

In one embodiment the detector is a spectrometer. Other detectors may also be used depending on the purpose.

The probe is essentially tube shaped and comprises a distal D end and a proximal end P. The distal end D is for insertion into a body cavity and the proximal P end is for connection to an energy source. The tube shaped probe is divided into two portions, wherein the elongated portion comprising the distal end is called L1 and the portion in the proximal end is called L2. The length of L1 is in the range of 150-300 mm and the length of L2 is in the range of 30-40 mm. In one embodiment, the length of L1 is 190 mm. In other embodiments the length of L1 can be 160, 170 or 180 mm depending on the system used. In yet another embodiment, the length of L1 can be 200, 210, 220, 230, 240 mm or more, also depending on the system used. In yet another embodiment, the length of L1 may be in the range of 250 to 300 mm, depending on the system used.

In yet another embodiment, the L1 portion is extendable to a desired and predetermined length.

The diameter d1 of L1 is in the range of 1.2 -2.8 mm, and the diameter d2 of L2 is in the range of 1.8-3.0 mm. In another embodiment the diameter d1 of L1 is in the range of 1.5 to 2.2 mm. In yet another embodiment the diameter d1 of L1 is about 2-2.3 mm. In another embodiment the diameter d1 of L1 is 2.1 or 2.2 mm.

Another object of the present invention is to provide a real time system for localizing and identifying a harvesting site in a tumour.

The system comprises:
a probe (1) according to the one described above,
a means for measuring the microvascular perfusion and the total backscattered light intensity (TLI) in the normal tissue structure and in the tumour,
and reflected spectra (DRS),
a means for measuring the emitted light and recording the microcirculation, and
a control device.

The tumour site may in one embodiment be the brain, but other sites may also be possible.

In yet another embodiment the system further comprises at least one audio device transforming a signal of emitted fluorescence to an audible alarm corresponding to high or low fluorescence intensity.

In one embodiment the system further comprises an audio device transforming the laser signal to an audible alarm, i.e., high or rapidly alarm corresponding to a vessel warning.

In one embodiment the system may also comprise a visualisation system comprising MR images and the trajectory along which the probe is getting inserted.

The present disclosure further provides a method for localizing and identifying the best site for harvesting a biopsy in a tumour tissue. The method comprises localizing and identifying a tumour in for example a brain. The method comprises the steps of:
- administrating 5-ALA in a dose of 5- 20 mg/kg to a subject prior to surgery,
- mounting a stereotactic device,
- drilling a hole at a predetermined site in a skull (predicted from the MR-images),
- moving the probe (1) to the pre drilled hole via a navigation system or by an electrical or hand driven mechanical device, or by hand,
- deploying the probe into the pre drilled hole with or without a supporting device,
- measuring and recording the microvascular perfusion, TLI and fluorescence stepwise with or without an electrical or mechanical insertion device or continuously along the way to the target,
- pushing the probe forward and record if the microcirculation is within a valid level meaning that no vessels are detected in front of the probe,
- determining where the biopsy is to be taken by measuring and recording emitted fluorescence light (every mm) before and throughout the tumour trajectory wherein the emitted fluorescence signal is illustrated as a peak or as an audio signal, where the high fluorescence signal indicates highly malignant tissue of said tumour, and finally
- removing the probe.

The signal may also be a light signal or a flash signal.

The amount 5-ALA may be in the range of 5-40 mg/kg body weight, usually it is 20 mg/kg body weight. 5 to 10 or 15 mg/kg body weight works well with the present invention.

The present system is beneficial for the patient as the surgical procedure is shortened due to reduced waiting time for pathological results in the OR, and biopsy samples are taken from a probable tumor sites. The introduction of LD as a "vessel warning" system increases the safety during surgery as hemorrhage is be prevented with the forward looking optical multiparametric probe.

### Brief description of the drawings

Fig. 1 Shows a schematic drawing of the probe (a) with multiparametric features for stereotactic optical biopsy and the configuration of the fibers (b).
Fig. 2 Shows a schematic drawing of the insertion trajectory to the tumour (left hand) and diagram of recorded LD backscattered light (TLI), microcirculation ("vessel warning") and fluorescence signal along the trajectory (right hand). The tumour is calculated to be around position 0 +/- several mm.
Fig. 3 Shows a diagram visualizing one single PplX fluorescence signal from a tumour tissue. The peak at 635 nm is an indicator of malignancy.
Fig. 4 Shows a schematic drawing of the system of the present invention, i.e., the LD and fluorescence system together with the probe, sound for vessel warning and tumour detection and image navigation.

### Detailed description of the invention

In the following, the present invention will be described in more detail by way of embodiments and with reference to the accompanying drawings.

Other features and uses of the invention and their associated advantages will be evident to a person skilled in the art upon reading the description and the examples.

It is to be understood that the present invention is not limited to the particular embodiments shown here. The following examples are provided for illustrative purposes and are not intended to limit the scope of the invention since the scope of the present invention is limited only by the appended claims.

If nothing else is defined, any terms and scientific terminology used herein are intended to have the meanings commonly understood by those of skill in the art to which this invention pertains. The term "about" as used in connection with a numerical value throughout the description and the claims denotes an interval of accuracy, familiar and acceptable to a person skilled in the art. Said interval is 10%.

The present invention provides an optical probe according to claim 1 with multiparametric features designed in order to comply with an optimal use in the operation room (OR) and the present routine procedures for biopsy harvesting at the neurosurgical department. The present disclosure combines fluorescence spectroscopy technique for identifying a fluorescence labelled marker in a tissue with a method for detecting vessels. The combination enables a safe and quick real-time stereotactic neurosurgical optical biopsy. Furthermore, the present invention further provides a unique possibility to investigate the microcirculation in brain tumour tissue and track vessels along insertion trajectory in order to reduce the risks for bleedings during the biopsy procedure.

The multiparametric feature enables detection of at least 4 signals at the same time, i.e. fluorescence, auto fluorescence, perfusion and TLI.

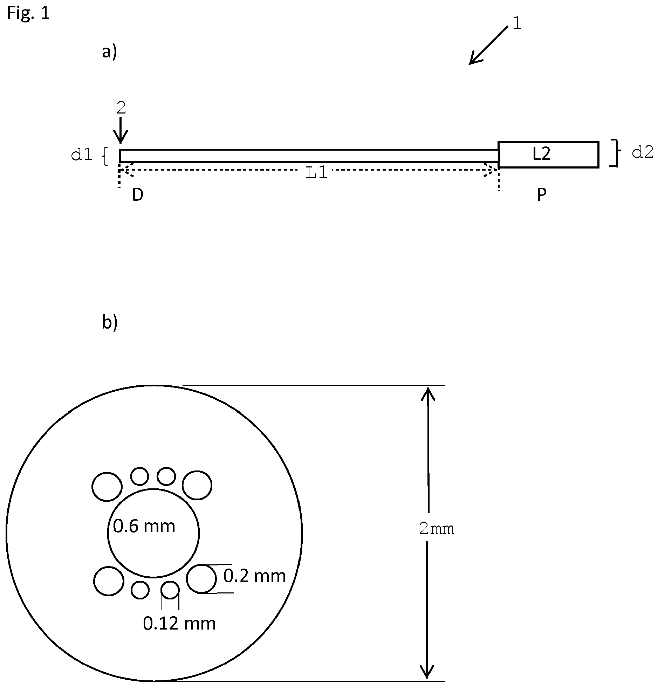

Fig. 1a shows a schematic drawing of the outer dimensions of the probe 1 of the present invention. The probe 1 is suitable to common commercial stereotactic and navigation systems used today, wherein some embodiments have a frame and others have not. Examples of navigation systems are the Leksell Stereotactic System (LSS), Brown Robert Wells (BRW), Richert-Mundinger (RM) or any other commercial frameless systems. Extern navigation systems using for example ultrasound or infrared indications can also be used in combination with the present invention.

The probe 1 is essentially tube shaped; having a distal D end for insertion into a body cavity and a proximal P end for connection to an energy source. The probe 1 may taper in the distal end D. The distal end D comprises a tip 2, which is the end of the probe that enters the tissue. The tip edge is in this embodiment slightly rounded, but can be blunter or more pointed in other embodiments. The total length of L1 in this embodiment is about 190 mm long, and the length of L2 is about 35 mm long. The dimensions of the probe may vary depending on the stereotactic system or navigation system used, the length of L1 may be in the range of 150-300 mm, and the length of L2 may be in the range of 30-40 mm. The diameter d2 of L2- is wider than the diameter d1 of L1. In this embodiment the diameter d2 of L2 is about 2.5 mm, and the diameter d1 of L1 is about 2-2.2 mm. The tip 2 of L1 is in some embodiments somewhat tapering, thus comprising a wider portion. The probe is connected to an energy source via the proximal end P of the probe. Along the inner shaft of L1, optical fibers are aligned towards the smoothly rounded tip. The fiber configurations (Fig. 1b) are in the range of 100-1000 µm depending on the function. The probe includes fluorescence emission (Fl em) and excitation (Fl exc) fibers, laser Doppler (LD) fibers and fibers for comprising diffuse reflection spectroscopy (DRS).

The fibers are connected to both the fluorescence system (Haj-Hosseini et al., 2010) and the LD modified for measurement of intracerebral microvascular blood flow and total light intensity (TLI) corresponding to tissue greyness (Wårdell et al., 2013). The probe is sterilized according to the Sterrad protocol and has a long cable in order to fulfil the safety requirements in the OR.

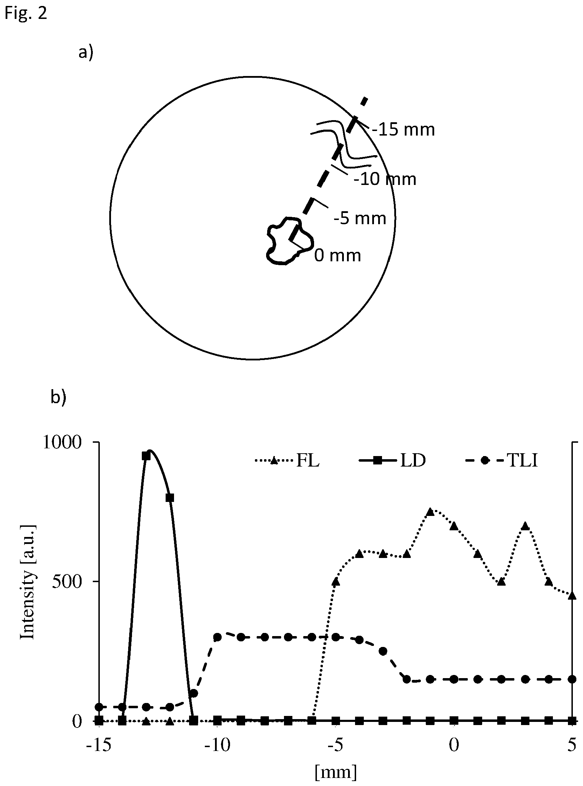

A schematic view of the insertion trajectory to the tumour is shown in Fig. 2a. Fig 2b shows an example of a diagram on the recorded LD backscattered light (TLI), perfusion (microcirculation) and fluorescence signal along the trajectory. The tumour is calculated to be around position 0 +/- several mm and a small vessel at X mm.

### Stereotactic biopsy procedure using the optical multiparametric probe

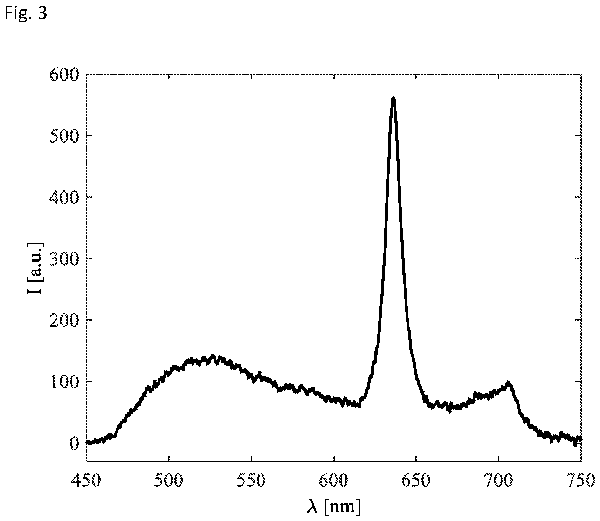

Prior to surgery the patient is given a 5-20 mg/kg dose of 5-ALA, and the stereotactic frame is attached to the patient's head before MRI. Target and the trajectories towards the tumour are calculated by use of surgical planning software (e.g., Surgiplan, Elekta AB) and transformed to the co-ordinates of the stereotactic system. After skull opening, the optical probe is inserted in 1 mm steps with the mechanical device by the surgeon, from cortex towards the tumour core. Each step is followed by a recording of the microcirculation and TLI with the LD. When approaching the suspected lesion, the fluorescence of PpIX is measured and in case of tumour tissue a fluorescence peak is immediately visible (Fig. 3). The peak at 635 nm is an indicator of malignancy. The data collection is done by the engineer in communication with the neurosurgeon. When the entire trajectory has been passed, the optical probe is retracted and replaced by the biopsy needle which is carefully inserted towards the suspected lesion. Biopsies are then taken according to the clinics' routine, i.e. at 3-5 positions along the trajectory. By using the mechanical device, the exact position for the optical recordings and biopsies can be defined and matched also to the preoperative MRI in the postoperative analysis of the collected data. It is also possible to stop the insertion if the blood flow indicates a vessel structure i.e. high blood flow in front of the probe tip (Fig. 2b).

A few tissue samples are immediately sent to the pathological department for analysis, and the results must be received before the surgical procedure can be closed. In case the biopsies do not show diagnostic results the surgery needs to be proceeded for an additional tissue harvesting along a second trajectory. The waiting time is usually about one hour, and this time and the re-doing of the operation can be avoided by the optical biopsy which gives the results in an instance and guides the surgeon in harvesting the biopsies from the most probable tumor locations. Other samples are sent for more accurate postoperative analysis. The samples are subsequently examined under the microscope determining the presence of the type of malignancy and its grade (Louis et al., 2007). A postoperative CT or MRI is done to monitor the bleedings. The average microcirculation and TLI as well as the fluorescence peaks are plotted against the anatomical position along the trajectory, and the corresponding biopsy results.

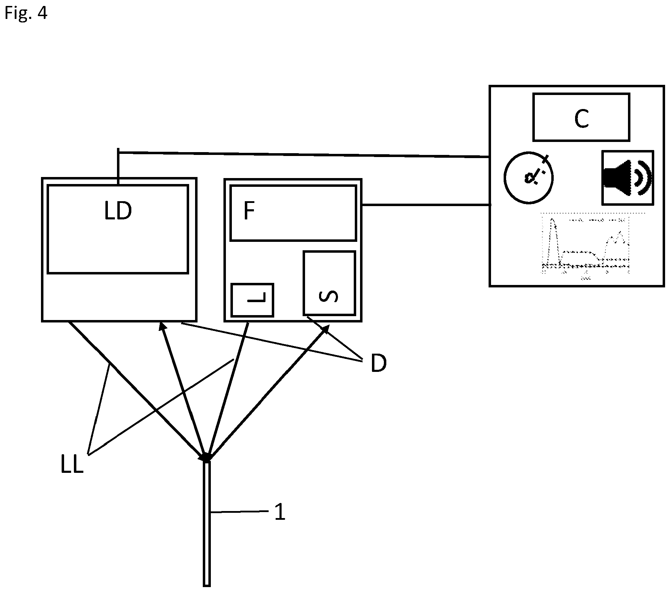

A schematic drawing of the system of the present invention comprising the LD and fluorescence system together with the probe, sound and image navigation are shown in Fig. 4.

### Preliminary results

By using highly sensitive measurement techniques as disclosed in the present disclosure the biopsy is indeed performed in malignant tissue of the tumour, and ensures that the method of obtaining a biopsy is significantly improved.

Applications of this method and complementary optical methods have benefits for diagnosis during stereotactic biopsy and thus improvement of the surgical procedure. An on-line optical signature of malignant tissue as seen through the PpIX-peak is beneficial for the patient and health care in general as the surgical procedure could be shortened due to the reduced waiting time for pathological results and the biopsy sampling can be placed with a significantly higher precision. The introduction of laser Doppler (LD) flowmetry as a vessel warning system increases the safety during surgery as hemorrhage could be prevented with the forward looking probe. By combining these features in the same surgical session two important tasks can be achieved which are beneficial for the patient.

The present invention discloses a novel probe with multiparametric features for localizing and identifying an appropriate site for harvesting a biopsy prior to stereotactic optical biopsy. With the expression appropriate site it is meant a site in the tumour tissue that reflects the malignity of the tumour thereby increasing the chance to correct diagnosis and treatment of the patient. The present invention counteracts some of the serious drawbacks, such as haemorrhage and misdiagnosis of malign tissue, of prior art. Even more important from a clinical perspective is the real-time analysis of the blood flow and fluorescence.

The probe is compatible with commercial navigation and monitoring systems used today. The present probe detects the surrounding tissue type more accurately due to its inventive combination of optical sources, thereby providing a superior probe.

### References

Haj-Hosseini, N., Richter, J., Andersson-Engels, S. & Wårdell, K. 2010. Optical touch pointer for fluorescence guided glioblastoma resection using 5-aminolevulinic acid. Lasers Surg Med, 42, 9-14.
Louis, D. N., Ohgakl, H. & Wiestler, O. 2007. WHO classification of tumours of the central nervous system, International Agency for Research on Cancer.
Pålsson, S., Backlund, E. O., Eriksson, O., Lundberg, P., Smedby, O., Sturnegk, P., Wårdell, K., Svanberg, K. & Andersson- Engels, S. 2003. ALA-PpIX fluoroscence and MR spectroscopy in connection with stereotactic biopsy of human glioblastomas. PhD-thesis, Lund Institute of Technology
Stummer, W., Pichlmeier, U., Meinel, T., Wiestler, O. D., Zanella, F. & Reulen, H.-J. 2006. Fluorescence-guided surgery with 5-aminolevulinic acid for resection of malignant glioma: a randomised controlled multicentre phase III trial. The Lancet Oncology, 7, 392.
Wårdell, K., Zsigmond, P., Richter, J. & Hemm, S. 2013. Relationship between laser Doppler signals and anatomy during deep brain stimulation electrode implantation toward the ventral intermediate nucleus and subthalamic nucleus. Neurosurgery, 72, ons127-40.

## Claims

1. An optical probe (1) for intracerebral guidance prior to stereotactic tumour biopsy comprising an essentially tube shaped outer housing accommodating optical fibres, said tube shaped outer housing being divided into a first and a second portion, wherein the first portion comprises a distal (D) end for insertion into a brain of a subject and the second portion comprises a proximal (P) end for connection to an energy source, the total length (L1) of the first portion is in the range of 150-300 mm and the length (L2) of the second portion is in the range of 30-40 mm, the diameter (d2) of the proximal end (P) is wider than the diameter (d1) of the distal end (D), wherein the optical fibres include fluorescence emission and excitation fibres and laser Doppler fibres, and wherein the optical fibres are arranged in close proximity towards the distal end (D), such that the probe is forward looking, and wherein the fibres are operatively connected via the proximal end (P) to a fluorescence system for localizing and identifying a site for harvesting a biopsy in a brain tumour tissue prior to stereotactic tumour biopsy, and to a laser doppler system for measuring intracerebral microvascular blood flow, wherein the fluorescence system includes a blue laser providing light at 405 nm for fluorescence excitation and the laser doppler system includes light source providing light at 780 nm.

2. The optical probe (1) according to claim 1, further comprising fibers for measuring diffuse reflection spectroscopy wherein the system includes a white light source providing light in a wavelength interval of 350-950 nm.

3. The optical probe (1) according to claim 1 or 2, wherein the length (L1) of the first portion is 190 mm.

4. The probe according to any of claim 1-3, wherein the diameter (d1) of the first portion is in the range of 1.2 -2.8 mm, and the diameter (d2) of the second portion is larger than the diameter of the first portion, preferably in the range of 1.2-5.0 mm.

5. A real time system for localizing and identifying the best site for harvesting a biopsy in a brain tumour tissue, said system comprising:
- an optical probe (1) according to any one of claims 1-4,
- a means for measuring and recording the microvascular perfusion and the total backscattered light intensity (TLI) in the normal brain structure and in the tumour, and reflected spectra (DRS),
- a means for measuring the emitted light and recording the microcirculation, and
- a control device.

6. The system according to claim 5, further comprising an analysis system.

7. The system according to any of claims 5 or 6, further comprising at least one audio device transforming a signal of emitted fluorescence to an audible alarm corresponding to low, medium and high fluorescence intensity, wherein the alarm corresponds to the intensity of said signals.

8. The system according to any of claims 5 to 7, further comprising an audio device transforming the laser signal to an audible alarm, i.e. high or rapidly alarm corresponding to a vessel warning.

9. The system according to any of claims 5 to 8, further comprising a visualisation system comprising MR images and the trajectory along which the probe is getting inserted.

## Patentansprüche

1. Optische Sonde (1) für eine intrazerebrale Führung vor einer stereotaktischen Tumorbiopsie, die ein im Wesentlichen röhrenförmiges Außengehäuse umfasst, das optische Fasern beherbergt, wobei das röhrenförmige Außengehäuse in einen ersten und einen zweiten Abschnitt unterteilt ist, wobei der erste Abschnitt ein distales (D) Ende für eine Einführung in ein Gehirn eines Subjekts umfasst und der zweite Abschnitt ein proximales (P) Ende für eine Verbindung mit einer Energiequelle umfasst, wobei die Gesamtlänge (L1) des ersten Abschnitts in dem Bereich von 150-300 mm liegt und die Länge (L2) des zweiten Abschnitts in dem Bereich von 30-40 mm liegt, wobei der Durchmesser (d2) des proximalen Endes (P) breiter als der Durchmesser (d1) des distalen Endes (D) ist, wobei die optischen Fasern Fluoreszenzemissions- und -anregungsfasern und Laser-Doppler-Fasern einschließen, und wobei die optischen Fasern in unmittelbarer Nähe zu dem distalen Ende (D) derart angeordnet sind, dass die Sonde nach vorne gerichtet ist, und wobei die Fasern über das proximale Ende (P) mit einem Fluoreszenzsystem zum Lokalisieren und Identifizieren einer Stelle zum Entnehmen einer Biopsie in einem Gehirntumorgewebe vor der stereotaktischen Tumorbiopsie und mit einem Laser-Doppler-System zum Messen von intrazerebralem mikrovaskulärem Blutfluss wirkverbunden sind, wobei das Fluoreszenzsystem einen blauen Laser einschließt, der Licht bei 405 nm für eine Fluoreszenzanregung bereitstellt, und das Laser-Doppler-System eine Lichtquelle einschließt, die Licht bei 780 nm bereitstellt.

2. Optische Sonde (1) nach Anspruch 1, die ferner Fasern zum Messen einer diffusen Reflexionsspektroskopie umfasst, wobei das System eine Weißlichtquelle einschließt, die Licht in einem Wellenlängenintervall von 350-950 nm bereitstellt.

3. Optische Sonde (1) nach Anspruch 1 oder 2, wobei die Länge (L1) des ersten Abschnitts 190 mm beträgt.

4. Sonde nach einem der Ansprüche 1-3, wobei der Durchmesser (d1) des ersten Abschnitts in dem Bereich von 1,2-2,8 mm liegt und der Durchmesser (d2) des zweiten Abschnitts größer als der Durchmesser des ersten Abschnitts, vorzugsweise in dem Bereich von 1,2-5,0 mm, ist.

5. Echtzeitsystem zum Lokalisieren und Identifizieren der besten Stelle zum Entnehmen einer Biopsie in einem Gehirntumorgewebe, wobei das System Folgendes umfasst:
- eine optische Sonde (1) nach einem der Ansprüche 1-4,
- ein Mittel zum Messen und Aufzeichnen der mikrovaskulären Perfusion und der Gesamtintensität des rückgestreuten Lichts (*total backscattered light intensity-* TLI) in der normalen Gehirnstruktur und in dem Tumor, und reflektierter Spektren (DRS),
- ein Mittel zum Messen des emittierten Lichts und Aufzeichnen der Mikrozirkulation, und
- eine Steuervorrichtung.

6. System nach Anspruch 5, das ferner ein Analysesystem umfasst.

7. System nach einem der Ansprüche 5 oder 6, das ferner wenigstens eine Audiovorrichtung umfasst, die ein Signal der emittierten Fluoreszenz in einen hörbaren Alarm umwandelt, der einer niedrigen, einer mittleren und einer hohen Fluoreszenzintensität entspricht, wobei der Alarm der Intensität der Signale entspricht.

8. System nach einem der Ansprüche 5 bis 7, das ferner eine Audiovorrichtung umfasst, die das Lasersignal in einen hörbaren Alarm umwandelt, d. h. einen hohen oder schnellen Alarm, der einer Gefäßwarnung entspricht.

9. System nach einem der Ansprüche 5 bis 8, das ferner ein Visualisierungssystem umfasst, das MR-Bilder und die Trajektorie umfasst, entlang der die Sonde eingeführt wird.

## Revendications

1. Sonde optique (1) pour le guidage intracérébral avant une biopsie stéréotaxique d'une tumeur, comprenant un boîtier externe essentiellement en forme de tube recevant des fibres optiques, ledit boîtier externe en forme de tube étant divisé en une première et une seconde partie, la première partie comprenant une extrémité distale (D) pour insertion dans un cerveau d'un sujet et la seconde partie comprenant une extrémité proximale (P) pour une connexion à une source d'énergie, la longueur totale (L1) de la première partie étant dans la plage de 150 à 300 mm et la longueur (L2) de la seconde partie étant dans la plage de 30 à 40 mm, le diamètre (d2) de l'extrémité proximale (P) étant plus large que le diamètre (d1) de l'extrémité distale (D), les fibres optiques comportant des fibres d'émission et d'excitation de fluorescence et des fibres laser Doppler, et les fibres optiques étant disposées à proximité immédiate vers l'extrémité distale (D), de telle sorte que la sonde est tournée vers l'avant, et les fibres étant reliées de manière fonctionnelle par l'intermédiaire de l'extrémité proximale (P) à un système de fluorescence pour localiser et identifier un site de prélèvement d'une biopsie dans un tissu de tumeur cérébrale avant la biopsie stéréotaxique de la tumeur, et à un système de laser Doppler pour mesurer le débit sanguin microvasculaire intracérébral, le système de fluorescence comportant un laser bleu fournissant de la lumière à 405 nm pour l'excitation de fluorescence et le système de laser Doppler comportant une source de lumière fournissant de la lumière à 780 nm.

2. Sonde optique (1) selon la revendication 1, comprenant en outre des fibres pour mesurer une spectroscopie en réflexion diffuse, le système comportant une source de lumière blanche fournissant de la lumière dans un intervalle de longueurs d'onde de 350 à 950 nm.

3. Sonde optique (1) selon la revendication 1 ou 2, dans laquelle la longueur (L1) de la première partie est de 190 mm.

4. Sonde selon l'une quelconque des revendications 1 à 3, dans laquelle le diamètre (d1) de la première partie est dans la plage de 1,2 à 2,8 mm, et le diamètre (d2) de la seconde partie est supérieur au diamètre de la première partie, de préférence dans la plage de 1,2 à 5,0 mm.

5. Système en temps réel pour localiser et identifier le meilleur site pour le prélèvement d'une biopsie dans un tissu de tumeur cérébrale, ledit système comprenant :
- une sonde optique (1) selon l'une quelconque des revendications 1 à 4,
- un moyen pour mesurer et enregistrer la perfusion microvasculaire et l'intensité lumineuse totale (TLI) rétrodiffusée dans la structure cérébrale normale et dans la tumeur, et les spectres réfléchis (DRS),
- un moyen pour mesurer la lumière émise et enregistrer la microcirculation, et
- un dispositif de commande.

6. Système selon la revendication 5, comprenant en outre un système d'analyse.

7. Système selon l'une quelconque des revendications 5 ou 6, comprenant en outre au moins un dispositif audio transformant un signal de fluorescence émis en une alarme sonore correspondant à une intensité de fluorescence faible, moyenne et élevée, l'alarme correspondant à l'intensité desdits signaux.

8. Système selon l'une quelconque des revendications 5 à 7, comprenant en outre un dispositif audio transformant le signal laser en une alarme sonore, c'est-à-dire une alarme élevée ou rapide correspondant à un avertissement de vaisseau.

9. Système selon l'une quelconque des revendications 5 à 8, comprenant en outre un système de visualisation comprenant des images IRM et la trajectoire le long de laquelle la sonde est insérée.
